# EUROPEAN PATENT SPECIFICATION

(11) **EP 4 326 203 B1**
(45) Date of publication and mention of the grant of the patent: **19.11.2025**
(21) Application number: 21723115.8
(22) Date of filing: 20.04.2021
(51) Int. Cl.: A61F 13/15, A61F 13/537, A61L 15/58, B32B 5/02, B32B 5/26, D04H 1/425, D04H 1/4374, D04H 1/56, D04H 1/60, D04H 1/732

(54) **FIBROUS LAYER WITH HOTMELT COATING COMPOSITION**
FASERSCHICHT MIT HEISSSCHMELZBESCHICHTUNGSZUSAMMENSETZUNG
COUCHE FIBREUSE AVEC COMPOSITION DE REVÊTEMENT THERMOFUSIBLE

(43) Date of publication of application: 28.02.2024
(73) Proprietor: The Procter & Gamble Company, Cincinnati, OH 45202 (US)
(72) Inventor: ERDEM, Gueltekin, Beijing 101312 (CN); LINDNER, Torsten, 65824 Schwalbach am Taunus (DE)
(74) Representative: P&G Patent Germany
(86) International application number: PCT/CN2021/088384
(87) International publication number: WO 2022/222030

(56) References cited:
- US-A1- 2005 148 261
- US-A1- 2017 021 589

## Description

### FIELD OF THE INVENTION

The invention relates to a fibrous layer having a hotmelt composition applied on at least one of its external sides. The fibrous layer may be in particular an airlaid layer or a nonwoven layer. The fibrous layer typically comprises cellulose fibers, optionally intermixed with synthetic fibers such as spunbond or meltblown fibers. These fibers tend to break or detach from the fibrous layer during high speed manufacturing, a phenomenon referred to as "dusting". The hotmelt coating composition reduces dusting by coating and binding the fibers at the surface of the fibrous layer. The hotmelt coating composition may optionally comprise a hydrophilic additive to improve the fluid handling properties of the layer. A laminate may be formed by combining the fibrous layer with a substrate layer. The fibrous layer or laminate may be used in absorbent articles, in particular as a fluid acquisition-distribution layer.

### BACKGROUND OF THE INVENTION

Fibrous layers are commonly used to make absorbent sheets, wipes and personal hygiene absorbent articles. Personal hygiene absorbent articles such as baby diapers or adult incontinence products are multi-layered products that comprise a topsheet on the wearer-facing side, a backsheet on the garment-facing side and an intermediate absorbent core. These articles further comprise a nonwoven acquisition layer directly under the topsheet. Apart from the backsheet, which is typically a fluid impermeable film, any of these layers may be or comprise a fibrous layer comprising a cellulose fibers and/or synthetic fibers. These fibrous layers are made at high speed as continuous webs before being individualized by cutting. The webs may be formed as an intermediate roll at a supplier for further converting in a complex product such as a diaper at a later stage (off-line process), or the continuous webs may be directly cut in individual fibrous layers and fed e.g. in a converting line for complex absorbent product such as diapers (on-line process).

During the making of the fibrous layers, the continuous webs are transferred at high speed through the production line, which may cause the breakage or loss of fibers, especially cellulose fibers. This breakage or separation process is referred as "dusting" and cause the accumulation of fibers or fiber debris on the production line, which has a negative impact on several aspects, such as health and safety of the workforce, contamination of the line tools and ultimately the finished products. It is known to treat the surface of fibrous layers susceptible to dusting with a latex to avoid this problem. For example, WO2012/158692A1 (P&G) discloses an airlaid sheet material comprising a latex coating on both surfaces thereof. However, one drawback of latex coating is that it can introduce odor issues, create process difficulties (drying step), also at the converting stage (build up at idlers). US2017/0021589 disclose a fibrous web structure comprising scrim layers to reduce release of lint. US2005/0148261A1 discloses nonwoven webs with reduced level of lints and slough by treating the surface of the nonwoven web with a small amount of meltblown fibers.

There is therefore a need for a cost-efficient and simple process to reduce dusting, while retaining or even improving the fluid handling property of the fibrous layer.

### SUMMARY OF THE INVENTION

According to the invention, it was found that at least one of the side of the fibrous layer can be coated by a hotmelt composition, wherein the hotmelt composition at least partially coats and binds the fibers at the surface of the fibrous layer. This hotmelt coating was found to greatly reduce dusting. The hotmelt composition can be sprayed at the surface of the fibrous layer to be coated. The hotmelt composition comprises at least 50% by weight of a metallocene-catalyzed polymer, which is a propylene-based copolymer. The hotmelt composition may be substantially free of tackifier (< 10 weight percent), so that it is not tacky at room temperature and thus does not attach to itself when formed into a roll, for example. The hotmelt composition may optionally comprise a hydrophilic melt additive, as discussed in the body of the description below. The fibrous layer may be attached e.g. by gluing to a substrate layer on the side of the fibrous layer opposed to the hotmelt composition. Alternatively, the fibrous layer may comprise a hotmelt composition of the invention on the surfaces of the first side and second side. The fibrous layer or laminate of the invention may be used as a fluid acquisition-distribution layer disposed between a topsheet and an absorbent core.

### BRIEF DESCRIPTION OF THE DRAWINGS

Fig. 1 is a SEM picture of the external surface of the fibrous layer, showing how the fibers are superficially coated with a hotmelt coating composition which has been sprayed on this surface;
Fig. 2 shows a schematic illustration of one way to manufacture of a laminate comprising a fibrous layer according to the invention;
Fig. 3 shows a schematical cross-section of an absorbent article incorporating a laminate comprising a fibrous layer of the invention as part of an acquisition-distribution system.

### Preamble

As used in the specification and in the claims, the term "comprising" may include the embodiments "consisting of and "consisting essentially of." The terms "comprise(s)," "include(s)," "having," "has," "can," "contain(s)," and variants thereof, as used herein, are intended to be openended transitional phrases, terms, or words that require the presence of the named ingredients/steps and permit the presence of other ingredients/steps. However, such description should be construed as also describing compositions or processes as "consisting of and "consisting essentially of the enumerated ingredients/steps, which allows the presence of only the named ingredients/steps, along with any impurities that might result therefrom, and excludes other ingredients/steps.

All % are by weight of the adhesive composition unless indicated otherwise.

### DETAILED DESCRIPTION OF THE INVENTION

### Hotmelt compositions

Hotmelt compositions are materials that are solid at room temperature and applied in the molten state to a substrate typically by spraying or slot coating. Hotmelt compositions have been used as adhesives to adhesively attach two layers in personal hygiene articles. As is known in the art, hotmelt compositions are made by combining one or more backbone polymers and additive components in a substantially uniform thermoplastic blend. Typical additive components include tackifiers, plasticizers, and/or waxes. Plasticizers such as mineral oil allows the hotmelt to be applied at lower temperature by reducing the viscosity of the composition. Tackifiers are compounds used to make hotmelt adhesives stick at room temperature.

The hotmelt composition of the invention on the other hand is not used as an adhesive between two layers, but is used to at least partially coats and binds the fibers at the surface of at least one side of a fibrous layer. The hotmelt composition of the invention thus does not attach the fibrous layer to another layer. The hotmelt composition is thus preferably substantially free (less than 10% by weight) and preferably free of tackifiers.

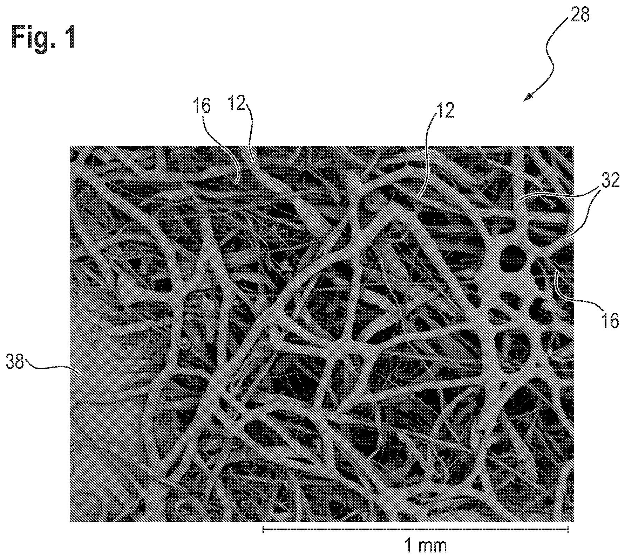

The hotmelt composition may be sprayed using any conventional hotmelt spray nozzle on the surface of the fibrous layer. Fig. 1 shows a SEM picture of the surface of a fibrous layer, which will be discussed in more details hereinafter, which has been sprayed with such a coating composition 32. As can be seen in Fig. 1, both cellulose fibers 12 and thinner, continuous meltblown fibers 16 are at least partially coated and bonded by the hotmelt composition 32.

Spraying a hotmelt fiber coating composition on the external surface of the fibrous layer was found to immobilize the cellulose and/or spunmelt fibers on this surface of the fibrous layer. This was found to reduce fibers from breaking off the fibrous layer and forming "dust" that contaminates the manufacturing lines, in particular the absorbent article manufacturing line where the fibrous laminate or a laminate containing it may be incorporated as an acquisition-distribution system. Hotmelt compositions according to the invention were found to be efficient at reducing dusting and further do not require a drying step, unlike latexes, as the hotmelt composition quickly pass from the molten state to the solid state when deposited on the fibers. The hotmelt compositions may comprise one or more hydrophilic melt additives, in particular those exemplified below, to improve the liquid transfer properties of the fibrous layer. This is particularly useful when the fibrous layer is to be used as a component of a personal hygiene article such as a diaper.

### mPO

It was found that metallocene-catalyzed polymers (mPO) are particularly suitable as backbone for the hotmelt composition. The hotmelt composition comprises from 50% to 100% by weight of metallocene-catalyzed polymer(s). A single metallocene-catalyzed polymer may be used, or alternatively blends of metallocene-catalyzed polymers. Thus, unless indicated otherwise, when using the term "a metallocene-catalyzed polymer" it is meant the "one or more metallocene-catalyzed polymer(s)".

The hotmelt composition of the invention may in particular comprise one or more low molecular weight metallocene-catalyzed polymer having a peak molecular weight ranging from 10,000 g/mol to 130,000 g/mol. The peak molecular weight is measured according to the Peak Molecular Weight (Mp) Measurement Method disclosed further below.

Metallocene-catalyzed polymers typically have a regular spatial repeat monomer unit distribution and a narrow molecular weight distribution, as is known in the art. The suitable propylene-based metallocene-catalyzed polymers of the invention are copolymers, in particular propylene-ethylene copolymers. The propylene-ethylene copolymers comprise at least 50% by weight of the copolymer of propylene unit, in particular at least 60%, or at least 70%, or at least 80% by weight. The remaining monomers are ethylene monomers, and optionally other alpha olefin monomers may be present in the co-polymers, for example 4-methyl-1-pentene, pentene-1, 2-methylpentene-1, 3-methylbutene-1, heptene-1, dimethylpentene-1, trimethylbutene-1, ethylpentene-1, methylpentene-1, trimethylpentene-1, methylethylpentene-1, 1-octene, diethylbutene-1, propylpentane-1, decene-1, methylnonene-1, nonene-1, trimethylheptene-1, methylethylbutene-1, dodecene-1, and hexadodecene-1, and combinations thereof. The exact monomer distribution is typically published by the supplier, but can also be determined by a suitable method, such as nuclear magnetic resonance or infrared spectroscopies.

Suitable metallocene-catalyzed propylene-ethylene copolymers are commercially available from Clariant under the trade name Licocene^{®}, with a broad range of properties such as molecular weight, viscosity, crystallinity, etc... US2016/053,149A1 assigned to Clariant also describes suitable co-polymers and on page 5 indicates that these examples were produced by the processes indicated in EP571,882. For a given catalyst system and given comonomer ratio, the molecular weight was regulated via the hydrogen partial pressure as molar mass regulator.

The hotmelt composition may comprise a blend of two polymers having different molecular weight, in particular a low molecular weight metallocene-catalyzed propylene-ethylene copolymer having a peak molecular weight ranging from 10,000 g/mol to 130,000 g/mol, as indicated above, and a higher molecular weight polymer.

A commercial example of the low molecular weight copolymer is Licocene^{®} PP 1602 from Clariant. Licocene PP 1602 is sold as granules and is described as a low melting, metallocene-technology based propylene-ethylene copolymer, which exhibits a low degree of crystallinity. The Mp of Licocene^{®} PP 1602 was measured to be 75,900 g/mol. Another example is Licocene^{®} PP 1302. The Mp of Licocene^{®} PP 1302 was measured to be 24,100 g/mol. Licocene^{®} PP 3602 which is sold as granules and is described as a low crystalline metallocene-catalyzed propylene-ethylene copolymer may also be used. Further examples of low molecular weight polymers are Vistamaxx 8780 and Vistamaxx 8880 from Exxon. A blend of two or more low molecular weight metallocene-catalyzed propylene-ethylene copolymers may be used, as blending two lower molecular weight copolymers with different crystallinity was found to enable low stiffness while still maintaining high Toughness. An example is a blend of Licocene^{®} 3602 and Licocene^{®} 1602, which are both propylene-ethylene copolymers from Clariant. Licocene 3602 is a relatively highly crystalline polymer while Licocene 1602 has a medium crystallinity. In a blend of both (e.g. 2:1 for the ratio of Licocene 1602 to Licocene 3602), the overall crystallinity can be adjusted in a way that the resulting hotmelt composition provide good anchoring points of the cellulose fibers. Another example is a 2:1 blend of Licocene 1602 and Licocene 2602.

The Toughness of the formulation can be significantly increased when a polyolefin having a high peak molecular weight Mp of from 130,000 g/mol to 700,000 g/mol, is added in the formulation. The high molecular weight polyolefin may have a peak molecular weight which is at least greater by 10,000 g/mol than the peak molecular weight of the low molecular weight metallocene-catalyzed polyolefin(s) described above (the highest peak for blends), in particular at least 20,000 g/mol, or even at least 50,000 g/mol greater. The high molecular weight polyolefin may in particular have a peak molecular weight of from 140,000 g/mol to 410,000 g/mol, or from 150,000 g/mol to 360,000 g/mol.

The inventors have found that, surprisingly, the addition of a longer molecular weight polyolefin significantly increases the strain hardening of the blend besides increasing the elongation at break, which in combination results in a significantly higher Toughness of the formulation. Strain hardening is believed to be a "self-repairing mechanism of the blend when being strained, which avoids early rupture.

The higher molecular weight polymer, if present, may be advantageously comprised of a single material to simplify the compounding and formulation of the hotmelt composition but it may also be a blend of individual polymers. The hotmelt composition may for example comprise from 1% to 20% of such a high molecular weight polymer(s), by weight of the hotmelt composition. Already small additions of the longer molecular weight polymers may boost the strain hardening and hence the Toughness of the hotmelt composition. More than 10% by weight may on the other hand increase the viscosity. The higher molecular weight polyolefins may be a homopolymer or a copolymer. The copolymer may comprise different alpha olefin monomers such as ethylene, propylene, 4-methyl-1-pentene, pentene-1, 2-methylpentene-1, 3-methylbutene-1, heptene-1, dimethylpentene-1, trimethylbutene-1, ethylpentene-1, methylpentene-1, trimethylpentene-1, methylethylpentene-1, 1-octene, diethylbutene-1, propylpentane-1, decene-1, methylnonene-1, nonene-1, trimethylheptene-1, methylethylbutene-1, dodecene-1, and hexadodecene-1, and combinations thereof.

The higher molecular weight polymers may be in particular a propylene-ethylene copolymer. The high molecular weight polyolefin may also be a metallocene-catalyzed based copolymer, in particular a metallocene-catalyzed propylene-ethylene copolymer. The high molecular weight polyolefin may in particular be a propylene-ethylene copolymer comprising greater than 80 wt.% of polypropylene units with isotactic stereochemistry. Examples of such copolymers are commercially available as the Vistamaxx series from ExxonMobil. For example, Vistamaxx 6202 and Vistamaxx 6502 are sold as pellets and are described by their manufacturer as primarily composed of isotactic propylene repeat units with random ethylene distribution, produced using a metallocene catalyst technology. Vistamaxx 6202 and 6502 can be used as high molecular weight polymers in the formula examples below. Vistamaxx 6502 has the lowest viscosity, and thus the least impact on increasing the viscosity of the total composition.

Nonlimiting examples of commercially available higher molecular weight polymers are Affinity EG 8200G, Engage 8200, Infuse 9817, Vistamaxx 3000, Vistamaxx 6102, Vistamaxx 6202, Vistamaxx 6502, VERsify 4200, VERsify 4301. The higher molecular weight polymer may also be a block copolymer styrene-butadiene also known as styrene-butadiene copolymer (SBC). Kraton MD 1648 is a commercial example of SBC that may be used in the hotmelt composition of the present invention.

A hotmelt composition according to the invention may also be advantageously formulated with a single polymer that enables the desired properties of the laminate while reducing complexity of the formulation. A metallocene-catalyzed propylene-ethylene copolymer under the tradename Licocene PP 2502 having a peak molecular weight of 57,100 g/mol was found particularly suitable. Another commercial example of polymer that may be used as single polymer backbone for the hotmelt composition is Vistamaxx 8780, however it is less preferred due to the higher viscosity of Vistamaxx 8780.

### Optional components:

Optional components may be further added to the hotmelt composition, as is known in the art, in particular plasticizers, antioxidants, hydrophilic melt additives and the like. Tackifiers on the other hand are preferably reduced compared to conventional hotmelt adhesives, or completely eliminated from the composition. Tackifiers otherwise called "tackifier resins" or "tackifying resins" are low-molecular weight compounds (oligomers) that are added to adhesive formulations to improve tack and peel adhesion materials. Usual tackifiers known in the art may be used in the present invention. Typical tackifiers are thermoplastic materials stable at least up to 200 °C, being amorphous glasses at room temperature, and having a Tg higher than 50 °C, preferably comprised between 80 °C and 125 °C. Tackifiers typically have a molecular weight comprised between 500 and 2000 Daltons. Tackifiers are in general organic chemicals with polycyclic structure. Commonly used tackifiers are selected from rosin resins and their derivatives (rosin esters), hydrocarbon resins produced from petroleum-based by-products of naphtha crackers, and terpene resins (modified or not). Hydrocarbon resins may be aliphatic, cycloaliphatic and aromatic resins (in particular C5 aliphatic resins, C9 aromatic resins, and C5/C9 aliphatic/aromatic resins), and may be optionally hydrogenated hydrocarbon resins.

There are several advantages to hotmelt compositions with low amount or no tackifier present. Firstly, as indicated, this reduce the complexity of the formulation and for example facilitate quality control and traceability of the ingredients. Reducing or eliminating tackifiers in the hotmelt composition further allows forming a roll of the laminate 36 without the risk of the laminate sticking to itself.

### Antioxidant

The hotmelt composition may optionally comprise an antioxidant. Non-limiting examples of suitable antioxidants include amine-based antioxidants such as alkyl diphenyl amines, phenyl-naphthylamine, alkyl or aralkyl substituted phenyl-naphthylamine, alkylated p-phenylene diamines, tetramethyl-diaminodiphenylamine and the like; and hindered phenol compounds such as 2,6-di-t-butyl-4-methylphenol; 1,3,5-trimethyl-2,4,6-tris(3',5'-di-t-butyl-4'-hydroxybenzyl)benzene; tetra kis [(methylene(3,5-di-t-butyl-4-hydroxyhydrocinnamate)]methane (e.g., IRGANOXTM 1010, from Ciba Geigy, New York); octadecyl-3,5-di-t-butyl-4-hydroxycinnamate (e.g., IRGANOXTM 1076, commercially available from Ciba Geigy) and combinations thereof. When used, the amount of the antioxidant in the hotmelt composition can be respectively less than 1 %, alternatively from about 0.05 % to about 0.75 %, and alternatively from about 0.1 % to about 0.5 %, by weight of the hotmelt adhesive.

The hotmelt composition may optionally comprise a UV stabilizer that may prevent or reduce the degradation of the composition by radiation. Any UV stabilizer known to a person of ordinary skill in the art may be used in the hotmelt composition. Non-limiting examples of suitable UV stabilizers include benzophenones, benzotriazoles, aryl esters, oxanilides, acrylic esters, formamidine carbon black, hindered amines, nickel quenchers, hindered amines, phenolic antioxidants, metallic salts, zinc compounds, and combinations thereof. Where used, the amount of the UV stabilizer in the hotmelt composition can be less than 1 %, alternatively from about 0.05 % to about 0.75 %, and alternatively from about 0.1 % to about 0.5 %, by weight of the hotmelt composition.

The hotmelt composition may optionally comprise a fragrance such as a perfume or other odorant. Such fragrances may be retained by a liner or contained in release agents such as microcapsules that may, for example, release fragrance upon removal of a release liner from or compression on the adhesive composition. Where used, the amount of the fragrance in the hotmelt composition can be less than 3 %, alternatively less than 2 %, alternatively less than 1 %, alternatively from about 0.05 % to about 0.75 %, and alternatively from about 0.1 % to about 0.5 %, by weight of the hotmelt composition.

### Hydrophilic additive

The hotmelt composition may optionally comprise a hydrophilic additive. As for the other components indicated, the hotmelt composition may comprise one, two or more, of such hydrophilic additives. Hydrophilic melt additives are amphiphilic molecule having a hydrophilic head and a hydrophobic tail. The hydrophilic head is oriented towards the surface of the adhesive, thus providing for the hydrophilic character of the hotmelt composition while the hydrophobic head remains in the polymer matrix. The hotmelt composition may have particularly stable hydrophilic properties, enabled by the compatibility of the metallocene-catalyzed polyolefin and the hydrophilic melt additive.

Hydrophilic melt additives are typically compounded in a masterbatch in the form of pellets than can be incorporated by homogenous mixing in the molten polyolefin. Commercial examples of hotmelt additives particularly compatible with a propylene-based metallocene-catalyzed polyolefin are PPM 15560 from Techmer (hydrophilic PP masterbatch) and Brij S2 (Croda). Further, in order of declining preference, Brij S10 (from Croda,) Unithox 450, Unithox 720 and Unithox 750 (from Baker Hughes) can be used. PPM 15560 is preferably used in a dosage of 0.5 weight percent of the masterbatch, Brij S2 and Brij S 10 in a dosage of preferably 2 weight percent of the active.Techsurf^{®} melt additives from Techmer have been used to impart hydrophilicity to polyolefin fibers, nonwoven fabrics, and specialty plastic applications, and are useful in the present invention.

US6,146,757 discloses a hydrophilic melt additive comprising a blend of a first wetting agent and a second wetting agent. The first wetting agent is at least one water insoluble nonionic alkoxylated alkyl phenol, and the second wetting agent is at least one compound selected from the group consisting of an alkoxylated fatty alcohol and a water-soluble, nonionic, nonhydrolyzable polyoxyalkylene-modified organosilicone polymer. While not wishing to be bound by theory, it is believed that Techmer PPM 15560 is a melt additive according to this formula, in particular wherein the first wetting agent is a an ethoxylated nonylphenol having about 4 moles of ethylene oxide and the second wetting agent is a water-soluble, nonionic, non-hydrolyzable polyoxyalkylene-modified organosilicone polymer. However, this example is not limiting the present invention, which can be reduced in practice with other melt additives, as exemplified above.

The additives of the Brij^{®} series from Croda are ethoxylated alcohols of the general formula: 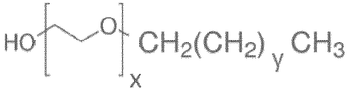 with x ranging from 2 to 100 and y ranging from 12 to 24, in particular y = 16 (stearyl).

For example, Brij S2 with x = 2 and y = 16 has a low molecular weight of 386 g/mol, which presumably facilitates the diffusion to the surface. These ethoxylated alcohols may be a more cost-effective alternative to the above mentioned blend. The blends described in US6,146,757 were found to enable a stronger hydrophilic effect, while Brij S2 enables a milder hydrophilic effect. One or the other additive may be thus preferred depending on the application purpose. In some applications (such as bonding of the topsheet to the acquisition layer), a milder hydrophilicity may be preferred to facilitate the draining of the top layer by lower layers in the diaper, and avoid liquid remaining in the top layer being exposed to the consumer.

A low contact angle is desirable so that water, urine or other water-based discharges "wet out" rather than "bead up" resulting in the fluid being directed into the absorbent core and away from the topsheet. The hydrophilicity of a hotmelt composition can be quantified by the Contact Angle Method described hereinafter. The hotmelt compositions can thus, if desired, exhibit a Fresh Film Contact Angle and an Aged Film Contact Angle with distilled water of less than 70 degrees, preferably of less than 50 degrees, as measured by the Contact Angle Test described herein.

### Properties

The hotmelt composition may typically have a storage modulus (G') higher than 10 MPa at 37°C, or even 15 MPa at 37°C. The storage modulus (G') may on the other be typically lower than 200 MPa at 37°C. The hotmelt composition has preferably a Storage Modulus (G') at 37°C between 3 MPa and 40 MPa.

The hotmelt composition may also have a storage modulus higher than 0.3 MPa at 23°C, or even higher than 15 MPa at 23°C, or even higher than 20 MPa at 23°C. On the other hand, the hotmelt material may also have a storage modulus that is lower than 300 x MPa at 23°C. Typically the G' value of polymers decreases when the temperature increases.

Commercially available propylene-ethylene copolymers, such as those from Clariant's Licocene, have the following G', as measured as indicated below:

| Hot Melt Composition | Storage modulus (G') at 37°C (MPa) | Storage modulus (G') at 23°C (MPa) |
|---|---|---|
| Licocene PP 1502 | 10 | 14 |
| Licocene PP 1602 | 8.6 | 12 |
| Licocene PP 2502 | 33 | 52 |
| L-MODU S 410 (PP homopolymer ex. Idemitsu) | 9.1 | 14 |
| Blend of 90% Licocene 2502 and 10% Vistamaxx 6502 | 26 | - |
| Blend of 95% Licocene 2502 and 5% Vistamaxx 6502 | 29 | - |
| Blend of 90% Licocene 2502 and 10% of the hydrogenated tackifier Eastotac Resh H-100L | 20 | - |

The hotmelt adhesive may also have a storage modulus (G') higher than 0.3 MPa at 60°C.

### Viscosity

The hotmelt composition should be processable at a temperature sufficiently low to avoid damaging the cellulose fibers of the fibrous layer for the selected processing application type, such as spray. Hotmelt compositions having a viscosity at 170°C ranging from 100 mPa.s to 10,000 mPa.s may thus be preferred. Hotmelt composition comprising a polymer backbone having a moderate chain length and molecular weight may thus be particularly suitable.

The table provides viscosities at 170°C for one-polymer-component hotmelt materials suitable for the present invention:

| Hotmelt Material | Viscosity [mPas] at 170°C |
|---|---|
| Licocene PP 2502 | 1,900 |
| Licocene PP 1602 | 5,900 |
| Licocene PP 2602 | 6,800 |

The following table provides viscosities at 150°C of further hotmelt materials suitable for the present invention, and estimated values at 170°C:

| Hot Melt Material | Viscosity [mPas] at 150°C | Viscosity [mPas] at 170°C (estimated) |
|---|---|---|
| 66.6% Licocene 1602 / 33.3% Licocene 2602 | 9,000 | 4,500 |
| 90% Licocene 2502 / 10 % Vistamaxx 6502 | 9,670 | 4,800 |
| 95% Licocene 2502 / 5 % Vistamaxx 6502 | 6,000 | 3,000 |
| 90% Licocene 2502 / 10 % Tackifier Eastotac Resh H-100L | 3,360 | 1,680 |

### Toughness

The hotmelt compositions of the invention may be further characterized by a Toughness of at least at least 10 MJ m⁻³, in particular at least 15 MJ m⁻³ as measured by the Extensional Test Method at 23 °C described further below. While not wishing to be bound by theory, the inventors believe that compositions having a relatively high Toughness provide a stronger coating of the fibers at the surface of the fibrous layer, thus further reducing the risk of dusting. The table below shows the values measured for various tackifier-free compositions. Licocene designates a range of metallocene-technology based propylene-ethylene copolymer. L-MODU designates a range of metallocene catalyst homopolymer polypropylene from Idemitsu. RT designates a range of Amorphous Poly Alpha Olefin (APAO) from Rextac.

| **Polymer** | **Source** | **Toughness [MJm⁻³]** | **Yield Stress [MPa]** |
|---|---|---|---|
| Licocene PP 2502 | Clariant | 28.0 | 9.0 |
| L-MODU S410 | Idemitsu | 84.7 | 7.5 |
| Licocene PP 1602 | Clariant | 26.1 | 4.7 |
| RT2830 | Rextac | 22.9 | 2.9 |
| Licocene PP 1302 | Clariant | 2.9 | 4.8 |
| Licocene PP 1502 | Clariant | 9.1 | 2.5 |

The hotmelt compositions of the invention may advantageously have a Yield Stress of at least 5 MPa. It is believed that Toughness and Yield Stress are corelated to the cohesive strength of the hotmelt. Higher values being linked to more deformation resistance for the hotmelt coating

### Fibrous layer 28

The present invention is applicable to any types of fibrous layers, in particular but not limited to airlaid fibrous layer. "Airlaid" refers to a web of fibers that is formed by dispersing fibers in an air stream and condensing them from the air stream onto a moving screen or substrate by means of a pressure or vacuum. Airlaids typically comprise cellulose fibers as main component (by weight) and may be mixed with other fibers, in particular synthetic fibers such as meltblown, spunmelt, or bicomponent fibers. Various airlaid processes and compositions are known in the art, the invention not being limited to a particular process or composition. However the invention is not limited to a fibrous layer obtained by an airlaid process, but is applicable to any type of nonwovens as well as wetlaids.

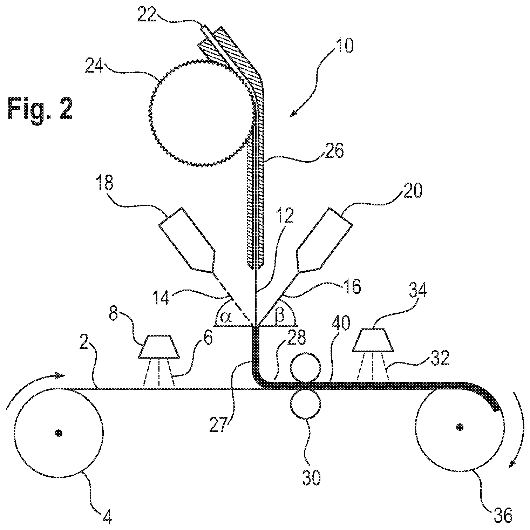

An exemplary fibrous layer deposition station 10 is depicted in Fig. 2. The fibrous layer 28 in this example is formed by airlaying cellulose fibers 12, optionally intermixed during the deposition process with other fibers, or materials 14, 16. The additional materials 14, 16 may be meltblown fibers, as is known in the art to make composite layer designed as "co-form" or "spinform". In the known spinform process, two streams of meltblown fibers are blown on each side of a central stream of cellulose fibers, where the fibers intermix, as disclosed for example in WO2020147231A1, WO2020/147232A1 (Xiamen Yanjan). The meltblown fibers are polypropylene fibers that are formed into very long and thin fibers (below 10 micrometer diameter). Meltblown fibers 16 are visible in Fig. 1 mixed with cellulose fibers 12 for example. Either of the meltblown nozzles may be used to spray a hotmelt material 14 into the stream of cellulose fibers 12 instead of spinning long, thin polypropylene meltblown fibers. The hotmelt material 14 may be the same or similar as the hotmelt coating composition 32 discussed above. This hotmelt 14 may improve the integrity of the fibrous web, by forming irregularly shaped lumps of hotmelt material that binds the fibers together.

The cellulose fibers 12 may be typically obtained from wood pulp 22, which is loosened through the loosening roller 24 and passed through the spout 26 under the action of an auxiliary air flow to form a stream of cellulose fibers 12. Alternatively, or additionally, chemically cross-linked cellulose fibers may also be used. Chemically cross-linked cellulose fibers have been used as distribution layer material, as for example disclosed in US2013/331806 (Rosati et al.). PE powders may also be introduced in the cellulose fibers stream. Other fibers, such as synthetic fibers or other plant-origin fibers, may also be mixed with the cellulose fibers (including cross-linked cellulose fibers). It is also possible to mix superabsorbent particles with the cellulose fibers, as it is disclosed in WO2020/147232A1 (Yanjan) in the context of wipes, to provide absorbency properties to the layer. Alternatively, the fibrous layer and the laminate may be free of superabsorbent particles. The cellulose fibers 12, including any chemically cross-linked cellulose fibers, are however typically present in amount greater than 50% by weight of the total weight of the fibrous layer 28.

The deposition station 10 may optionally comprise a second meltblown die 20 disposed downstream of the stream 12 of cellulose fibers, so that the meltblown fibers are at least partially intermixed with the cellulose fibers on this second side of the fibrous layer. The deposition station may thus comprise two meltblown dies, one on each side of the cellulose chute.

The fibrous layer 28 may thus optionally comprise meltblown fibers 16 made from a polymeric material, typically polypropylene, which is heated in the meltblowing process. The dissolved fine stream of thermoplastic resin is then ejected from the spinneret plates 20. The meltblown fibers are blown using a high temperature, high velocity hot gas stream into bundles of fibers having a fiber diameter < 10 µm. While meltblown fibers were used in the examples, spunbond fibers may also be introduced instead or in addition to the meltblown fibers 16. Spunmelt fibers are used herein means spunbond or meltblown fibers, as is known in the art. The projected hotmelt material 14 and if present the spunmelt fibers 16 (in particular meltblown fibers) are formed with a hot gas stream, and they each intersect one side of the cellulose pulp fibers stream 12 to form a combined fibrous stream 27 which deposits as a multilayer structural fibrous layer 28 on the substrate layer 2. The meltblown fibers 16, if present, typically consists of a single component having a high melting point. Other fibers may also be included additionally or alternatively in the stream of cellulose fibers, such as bicomponent fibers or low melting polyester fibers.

In the fibrous layer 28, the weight percent of cellulose fibers 12 is typically greater than 50% of the total weight of the fibrous layer 28, typically between 65% and 80% of the total weight of the fibrous layer. The fibrous layer may optionally comprise between 1% and 20% by weight of the hotmelt material forming the lumps of hotmelt material 17. The fibrous layer may also comprise between 10% and 30% by weight of the spunmelt fibers (meltblown and/or spunmelt), when present.

The combined stream 27 of cellulose fibers and additional material or fibers may be deposited on a substrate layer 2. As shown in Fig. 2, a substrate layer 2 is provided in a continuous manner, typically by unwinding a roll 4 of the substrate layer. The substrate layer is typically a nonwoven layer, for example an air-through bonded carded nonwoven. The substrate is advantageously hydrophilic, in particular if the substrate is later used as the acquisition layer in an acquisition-distribution system. Hydrophilic treatment such as a surface coating with surfactant or other hydrophilic agent are typically made by the substrate supplier before the substrate layer is formed in the roll 4, as is known in the art. The substrate 2 may have a basis weight higher than 15 gsm to be able to provide sufficient permeability and void volume, for example between 20 gsm and 60 gsm. The nonwoven substrate may in particular comprise fibers that are at least 1.5 dpf (denier per filaments).

After unrolling, a first side of the substrate layer 2 may be applied with a conventional adhesive 6. This adhesive 6 may be sprayed from a glue spray nozzle 8 as the substrate layer is passed under the nozzle. The adhesive 6 may also be applied by coating stripes of adhesive using a contact applicator. This first side of the substrate layer will be referred herein as the internal side as it will face the fibrous layer in the laminate, and the opposite side of the substrate will be referred herein to as its external side. Likewise, the side of the fibrous layer 28 facing the substrate layer is referred herein as the internal side, and the opposite side of the fibrous layer is referred as the external side. The internal side of the substrate layer 2 thus faces the internal side of the fibrous layer 28 in the laminate 40. The substrate layer 2 and the unitary fibrous layer 28 may be attached to each other by the adhesive 6 disposed at their interface.

The optional adhesive 6 deposited on the internal side of the substrate layer may comprise typical adhesive ingredient, in particular a polymer backbone, a tackifier and optional additives such as a plasticizer and antioxidants. The deposited adhesive basis weight can range from 1 to 15 g/m², in particular from 2 to 5 g/m². The function of the adhesive is to allow for a better integrity of the two layers of the laminate at their interface, but similar function may be provided by a mechanical or heat post-treatment, such as embossing, or sufficient integrity at the surface of the layer may be provided by the hotmelt material 14. Thus, while the adhesive treatment is advantageous, it is not always required.

The laminate 28 may be further consolidated by passing the two layers between a pair of calendaring rolls 30 to provide for a more intimate contact between the layers. This can improve adhesion between the layers by the adhesive 6, if present. This calendaring step further improves the integrity of the laminate and reduce dust formation. At least one of the rolls 30 may be heated and optionally structured with protruding embossment features to form bonding points 38 by melting the spunmelt/meltblown fibers 16, if present, at the external surface of the fibrous layer. Total bonding area from such a bonding unit may range from 3 % to 15% and preferably from 6% to 12% to enable integrity benefit without compressing the laminate excessively and affect fluid handling performance due to dense bond areas. Alternatively, the calendaring rolls 30 may be replaced by an oven to provide heat energy to melt any thermo-fusible fibers, in particular bicomponent fibers, present in the fibrous layer (see WO2020/147231A1 for disclosure of exemplary suitable thermofusible fibers).

The external surface of the fibrous layer 28 is sprayed with a coating composition 32 that at least partially coats and binds the cellulose fibers and optional spunmelt fibers on this external side of the fibrous layer. The hotmelt coating composition 32 may be sprayed using any conventional spray nozzle 34. Such a coating composition 32 is visible on the SEM picture of Fig. 1.

Spraying a fiber coating composition on any of the external surface of the first and/or second sides was found to immobilize the cellulose and/or spunmelt fibers on this side of the fibrous layer. These fibers may otherwise break off the fibrous layer and form "dust" that contaminates the manufacturing lines, in particular the absorbent article manufacturing line where the laminate may be incorporated as an acquisition-distribution system.

The hotmelt coating composition 32 may be the same or different than the hotmelt material 14 optionally sprayed in the stream of cellulose fibers. The hotmelt composition 32 and hotmelt material 14 (if present) may comprise the same polymer as backbone, in particular the same metallocene-catalyzed polymer, and even be identical, which simplifies inventory.

### Examples and Dust Data:

Different laminates were formed as generally described in Fig. 2 and were sprayed with a hotmelt coating composition on their external surface opposed to the substrate layer (for the inventive examples) or left without an hotmelt coating (comparative examples). The laminates had the following compositions:

| | Substrate layer (2) | Glue (8) | Hotmelt (18) or PP | Pulp (12) | Spunmelt (16) | Laminate Basis Weight | Sprayed Coating composition (32) |
|---|---|---|---|---|---|---|---|
| Inventive example 1 | 30 gsm Z87G ATB | 2 gsm Henkel581 3U | 3 gsm PP meltblown | 45 gsm | 8 gsm PP meltblown | 88 gsm | 2 gsm Licocene 2502 |
| Inventive example 2 | 30 gsm Z87G ATB | 2 gsm Henkel581 3U | 4 gsm PP meltblown | 70 gsm | 13 gsm PP meltblown | 119 gsm | 2 gsm Licocene 2502 |
| Inventive example 3 | 20 gsm spunbond NW | 2 gsm Henkel581 3U | 3 gsm Licocene 2502 | 45 gsm | 8 gsm PP meltblown | 78 gsm | 2 gsm Licocene 2502 |
| Inventive example 4 | 20 gsm spunbond NW | 2 gsm Henkel581 3U | 5 gsm Licocene 2502 | 70 gsm | 12 gsm PP meltblown | 109 gsm | 2 gsm Licocene 2502 |

Z87G is an air-through bonded acquisition layer material available from Yanjan nonwovens. It comprises 60% PE/PET (2 dpf, denier per filament), and 40% PE/PET fibers (4 dpf). The hotmelt material was pure Licocene 2502 from Clariant, with 0.02% (by weight) Hostavin N30 added as antioxidant. The meltblown PP material comprise 3% by weight of Croda Brij S2 hydrophilic agent.

In order to assess the tendency of a material to generate dust, a pre-defined amount of material (here 90 mm wide) is unwound and run over an idler with a defined wrap angle (here 90 degree) at a given speed (here 100 m/min) so that a given surface of material is ran (here 90 m²). The dust generated due to friction between material and idler is collected and weighed for comparison.

The dust collected was as follows:

| | Dust [g] | Dust Reduction [%] |
|---|---|---|
| Comparative example 1 | 0.0832 | - |
| Inventive example 1 | 0.014 | 83 |
| Comparative example 2 | 0.2525 | - |
| Inventive example 2 | 0.0951 | 63 |
| Comparative example 3 | 0.1255 | - |
| Inventive example 3 | 0.0569 | 55 |
| Comparative example 4 | 0.1544 | - |
| Inventive example 4 | 0.1088 | 31 |

The inventive examples exhibited considerable dusting reduction compared to the same fibrous layer without the coating composition.

### Absorbent article 41

"Absorbent articles", as used herein, refers to personal hygiene products that are placed against or in proximity to the body of the wearer to absorb and contain the various exudates discharged from the body. Absorbent articles include baby diapers, training pants, adult incontinence undergarments, feminine hygiene products, disposable inserts for hybrid diapers, bed matt, changing matt and the like. As used herein, the term "body fluids" or "body exudates" includes, but is not limited to, urine, blood, vaginal discharges and fecal matter. These articles are typically disposable, meaning are not intended to be laundered or otherwise restored or reused as a hygienic article after a single use. Disposable absorbent articles typically comprise a liquid pervious topsheet, a liquid impervious backsheet and an absorbent core positioned between the topsheet and the backsheet. The laminate of the present invention may be used in such an absorbent article as an acquisition-distribution system between the topsheet and the absorbent core.

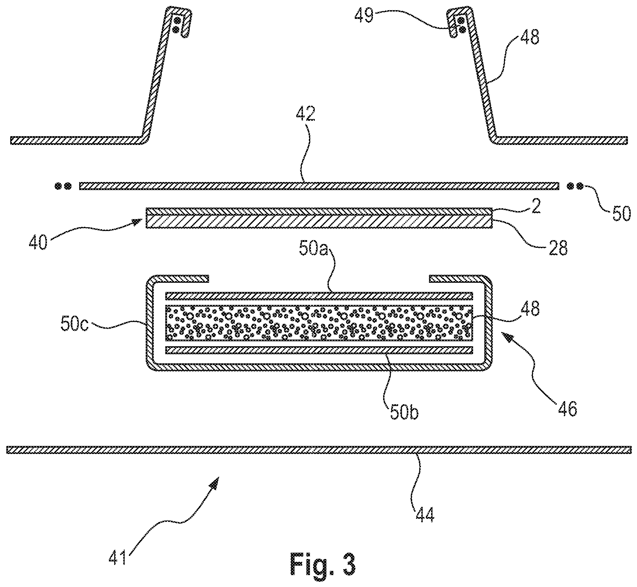

The fibrous layer 28 and/or the laminate 2 may be used as an acquisition-distribution system 40 of an absorbent article 41 such as a diaper, as exemplified in Fig. 3. The acquisition-distribution system is disposed between the topsheet 42 and the absorbent core 48. The acquisition-distribution system may be particularly used in combination with an absorbent core comprising no or low amount of cellulose fibers as absorbent material in the absorbent core.

The laminate of the fibrous layer may of course also be used for other applications, for example as a wipe for personal or surface cleaning, as a core wrap , in particular the top layer 50a of a core wrap.

Fig. 3 shows a schematic cross-section of a diaper 41 as exemplary absorbent article comprising a topsheet 42, backsheet 44 and absorbent core 46, which are discussed in more details below. The absorbent article 41 further comprises the laminate 40 of the invention as an absorption-distribution system disposed between the topsheet and the absorbent core. In this application, the substrate 2 is preferably a liquid permeable nonwoven, which may have been further treated by a hydrophilic agent to facilitate the acquisition of urine or other fluid from the topsheet. The fibrous layer 28 takes the role of a distribution layer, as the capillarity provided by the pores between the cellulose fibers can quickly acquire and distribute the fluid, passing it to the absorbent core. The hotmelt composition 32 may be applied on the external surface oriented towards the absorbent core 46, especially when the hotmelt composition comprises an hydrophilic additive.

In order to provide favorable fluid handling performance, the substrate layer 2 may have higher permeability (as measured according to industry standard test WSP 70.1 (20)) and lower liquid wicking rate (according to industry standard test WSP 10.1 (20)) when compared to the permeability and liquid wicking rate of the laminate as a whole.

A short description of the other components of a typical absorbent articles, in particular a taped or pant diapers now follows. The topsheet 42 is the part of the absorbent article 41 that is in contact with the wearer's skin. The topsheet may be joined to portions of the backsheet 44, the absorbent core 46, the barrier leg cuffs 48, and/or any other layers as is known to those of ordinary skill in the art. The topsheet may be compliant, soft-feeling, and non-irritating to the wearer's skin. Further, at least a portion of, or all of, the topsheet may be liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet may have one or more layers. The topsheet may be apertured, may have any suitable three-dimensional features, and/or may have a plurality of embossments (e.g., a bond pattern). The topsheet may be apertured by overbonding a material and then rupturing the overbonds through ring rolling, such as disclosed in U.S. Patent No. 5,628,097, to Benson et al., issued on May 13, 1997 and disclosed in U.S. Pat. Appl. Publication No. US 2016/0136014 to Arora et al. Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet.

The backsheet 44 is generally that portion of the absorbent article 41 positioned proximate to the garment-facing surface of the absorbent core 46. The backsheet may be joined to portions of the topsheet, the absorbent core 30, and/or any other layers of the absorbent article by any attachment methods known to those of skill in the art. The backsheet prevents, or at least inhibits, the bodily exudates absorbed and contained in the absorbent core from soiling articles such as bedsheets, undergarments, and/or clothing. The backsheet is typically liquid impermeable, or at least substantially liquid impermeable. The backsheet may, for example, be or comprise a thin plastic film, such as a thermoplastic film having a thickness of about 0.012 mm to about 0.051 mm. Other suitable backsheet materials may include breathable materials which permit vapors to escape from the absorbent article, while still preventing, or at least inhibiting, bodily exudates from passing through the backsheet.

The outer cover material (sometimes referred to as a backsheet nonwoven) may comprise one or more nonwoven materials joined to the backsheet 44 and that covers the backsheet 44. The outer cover material forms at least a portion of the garment-facing surface of the absorbent article and effectively "covers" the backsheet so that the film is not present on the garment-facing surface. The outer cover material may comprise a bond pattern, apertures, and/or three-dimensional features. The outer cover material may be a hydroentangled nonwoven material.

As used herein, the term "absorbent core" 46 refers to a component of the absorbent article 41 disposed in the article for absorbing and containing liquid such as urine received by the absorbent article. The absorbent core thus typically has a high absorbent capacity. The absorbent core comprises an absorbent material 48, that is typically enclosed within or sandwiched between a core wrap 50a, 50b.

The core wrap may be a single material that is folded and attached to itself, or it may comprise a separate top layer 50a and bottom layer 50b that may be bonded together. An overwrap 50c may also be present, especially for absorbent core comprising an absorbent layer substantially free of cellulose fibers. The absorbent material 48 typically comprises superabsorbent particles which are optionally mixed with cellulose fibers. As used herein, "absorbent core" does not include any acquisition-distribution systems, topsheet, or backsheet of the absorbent article.

The absorbent material 48 may be any conventional absorbent material known in the art. For example, the absorbent material may comprise a blend of cellulose fibers and superabsorbent particles ("SAP"), typically with the percentage of SAP ranging from about 50% to about 75% by weight of the absorbent material. The absorbent material may also be free of cellulose fibers, as is known in so-called airfelt-free cores, where the absorbent material consists of SAP. The absorbent material may also be a high internal phase emulsion (HIPE) absorbent foam.

"Superabsorbent polymer" or "SAP" refers herein to absorbent materials, typically cross-linked polymeric materials, that can absorb at least 10 times their weight of an aqueous 0.9% saline solution as measured using the Centrifuge Retention Capacity (CRC) test (EDANA method WSP 241.2.R3 (12)). The SAP may in particular have a CRC value of at least 20 g/g, in particular of from 20 g/g to 40 g/g. "Superabsorbent polymer particles", as used herein, refers to a superabsorbent polymer material which is in particulate form so as to be flowable in the dry state.

Various absorbent core designs comprising high amount of SAP have been proposed in the past, see for example in US5,599,335 (Goldman), EP1,447,066 (Busam), WO95/11652 (Tanzer), US2008/0312622A1 (Hundorf), WO2012/052172 (Van Malderen). In particular the SAP printing technology as disclosed in US2006/024433 (Blessing), US2008/0312617 and US2010/0051166A1 (both to Hundorf et al.) may be used. The invention is however not limited to a particular type of absorbent core. The absorbent core may also comprise one or more glue such as an auxiliary glue applied between the internal surface of one (or both) of the core wrap layers and the absorbent material to reduce leakage of SAP outside the core wrap. A micro-fibrous adhesive net may also be used in air-felt free cores as described in the above Hundorf references. These glues are not represented in the Figures for simplicity. Other core constructions comprising a high loft nonwoven substrate such as a carded nonwoven layer, having a porous structure into which SAP particles have been deposited, may also be used in present invention.

The basis weight (amount deposited per unit of surface) of the absorbent material may also be varied to create a profiled distribution of absorbent material, in particular in the longitudinal direction to provide more absorbency towards the center and the middle of the core, but also in the transversal direction, or both directions of the core. The absorbent core may also comprise one or more longitudinally extending channels, which are areas of the absorbent layer substantially free of absorbent material within the absorbent material layer. The top side of the core wrap may be advantageously bonded to the bottom side of the core by adhesive, mechanical or ultra-sonic bonding through these material-free areas. Exemplary disclosures of such channels in an airfelt-free core can be found in WO2012/170778 (Rosati et al.) and US2012/0312491 (Jackels). Channels may of course also be formed in absorbent cores comprising a mix of cellulose fibers and SAP particles. These channels can embody any suitable shapes and any suitable number of channels may be provided. In other instances, the absorbent core may be embossed to create the impression of channels. Absorbent cores with or without channels are also within the scope of the present disclosure.

The absorbent article 48 may comprise one or more pairs of barrier leg cuffs 48 and one or more pairs of leg elastics 50. The barrier leg cuffs 48 may be positioned laterally inboard of leg elastics 50. Each barrier leg cuff 48 may be formed by a piece of material which is bonded to the absorbent article 41 so it can extend upwards from a wearer-facing surface of the absorbent article and provide improved containment of body exudates approximately at the junction of the torso and legs of the wearer. The barrier leg cuffs 48 are delimited by a proximal edge joined directly or indirectly to the topsheet and/or the backsheet and a free terminal edge, which is intended to contact and form a seal with the wearer's skin. The barrier leg cuffs may extend at least partially between the front end edge and the back end edge of the absorbent article on opposite sides of the central longitudinal axis and may be at least present in the crotch region. The barrier leg cuffs 48 may each comprise one or more elastics 49 (e.g., elastic strands or strips) near or at the free terminal edge. These elastics 49 cause the barrier leg cuffs 48 to form a seal around the legs and torso of a wearer. The leg elastics 50 extend at least partially between the front end edge and the back end edge. The leg elastics 50 essentially cause portions of the absorbent article proximate to the chassis side edges to help form a seal around the legs of the wearer. The leg elastics may extend at least within the crotch region.

The absorbent articles may further comprise further common features for the type of absorbent articles, such as but not limited to, front and/or transversal barrier cuffs or waistbands, urine indicator that changes color when contacted with urine, fastening system with taped back ears and frontal landing zone, etc..

### Test Methods

### Viscosity Test Method

The Viscosity of a hot melt adhesive composition is determined by performing ASTM D3236-15 with the following additional guidance. A Brookfield RVT viscometer with spindle SC 4-27 (Brookfield Engineering, Middleboro, MA, USA), or equivalent, is used. The sample temperature is maintained at the desired temperature (170.0 ± 1.0 °C) throughout the measurement. The sample is preheated for 10 minutes and stirred with the measurement spindle for 30 min. The spindle is rotated at 20 rpm throughout the measurement. The resulting apparent viscosity, as described in section 10, is reported as the "viscosity" in units of millipascal-seconds to the nearest 100 mPa·s.

### Peak Molecular Weight (Mp) Measurement Method

The peak molecular weight is determined using a gel permeation chromatography (GPC) method. GPC is a well-known method wherein polymers are separated according to molecular size, the largest molecule eluting first. The peak molecular weights referred to herein can be determined with gel permeation chromatography (GPC) using polystyrene calibration standards, such as is done according to ASTM D5296. The molecular weight of any polymer or unknown polymer measured using GPC so calibrated is the styrene equivalent molecular weight, which herein is defined as the "peak molecular weight." Suitable solvents and temperatures are employed with GPC in order to achieve adequate molecular weight separation and resolution.

### Oscillatory Rheometry Test Method

The Oscillatory Rheometry Test Method is used to measure the Storage Modulus and the Loss Factor of a hotmelt composition. A controlled-stress rotational rheometer (such as Discovery HR-3, TA Instruments, New Castle, DE, USA, or equivalent) capable of sample temperature control (using a Peltier cooler and resistance heater combination) with a precision equal to or exceeding 0.5 °C over at least the range of -10 °C to 150 °C. The rheometer is operated in a parallel plate configuration with 20-mm stainless steel parallel-plate tooling.

A parallel plate gap of 1000 µm is initially used in the method. To compensate for thermal expansion of the tooling, the gap is set to 1000 µm, and a mapping of actual plate gap (as measured using a suitable standard test fluid) a function of temperature over the range -10 °C to 150 °C is performed. This mapping is then used throughout the determination of the Storage Modulus Parameter and the Loss Factor Parameter.

The rheometer is heated to 150 °C, hot melt adhesive composition is introduced in the rheometer, the gap is set to 1050 µm, excess protruding sample is trimmed, and the gap is then set to 1000 µm. (The axial force control of the rheometer is set to be maintained within ± 0.1 N of force, and the thermal expansion/contraction of the sample itself is compensated in order to avoid overfilling or underfilling of the gap in addition to the abovementioned compensation of the tooling.) The rheometer is then allowed to cool to 130 °C, at which point the measurement commences with temperature ramped from 130 °C to -10 °C at a constant rate of cooling of 2 °C/min. The applied strain amplitude is 0.1%, and the frequency of oscillation is 1 Hz (that is, one cycle per second). The resulting oscillatory stress is recorded.

After this step, the sample temperature is set to 23 °C (temperature is ramped to this setpoint at a rate of 10 °C/min), and the sample is allowed to rest for 4.0 hours at 23 °C. At the end of this period, the temperature is set to -10 °C (temperature is ramped to this setpoint at a rate of 10 °C/min), the sample is equilibrated for 300 seconds at -10 °C, and a second oscillatory rheology measurement is conducted (0.1% strain, frequency of oscillation of 1 Hz) while temperature is ramped upward to 130 °C at a constant rate of increase of 2 °C/min.

From the second, increasing temperature sweep, the storage modulus G' is calculated and recorded at 23 °C and 37 °C, and these values are reported in megapascals (MPa) to the nearest 0.01 MPa as the "Storage Modulus at 23 °C" and the "Storage Modulus at 37 °C," respectively. From the second, increasing temperature sweep, the loss factor (also known as tan delta) is calculated recorded at 23 °C and 37 °C, and these dimensionless values are reported to the nearest hundredth as the "Loss Factor at 23 °C" and the "Loss Factor at 37 °C," respectively.

### Extensional Test Method

The Extensional Test Method is used to determine the Yield Stress and the Toughness for a specimen of a polymer composition. A thin film specimen formed of polymer composition is analyzed with a rotational rheometer fitted with a specialized fixture with counter rotating rollers, and the stress associated with extensional strain imparted is measured and recorded.

### Instrumental setup

A rotational rheometer (ARES G2, TA Instruments, New Castle, DE, USA, or equivalent) is fitted with a fixture that has counter rotating cylindrical rollers specifically designed for the interrogation of extension deformation of films. An example of a suitable fixture is the Extensional Viscosity Fixture, or EVF (EVF, TA Instruments, or equivalent). The rheometer is further fitted with a forced-convection oven FCO (FCO, TA Instruments, or equivalent) and cooling system (ACS 2, TA Instruments, or equivalent) capable of controlling temperate from at least -50 to 250 °C to a within a tolerance of 0.5 °C.

### Specimen preparation

Approximately 6 g ± 2 g of the polymer composition is placed in a circular polytetrafluoroethane (PTFE) bowl with a flat bottom (diameter of 60 mm ± 2 mm) and introduced into a vacuum oven held at 170°C. After 15 minutes at ambient pressure, the pressure is lowered to 10 mbar, and the polymer composition is subsequently held at 170°C and at 10 mbar for 45 minutes to remove air bubbles from the polymer composition. If 170°C is insufficient to melt the polymer compositions a temperature 30 ± 10 °C above the melting temperature of the polymer material composition is used. The polymer composition is removed from the vacuum oven and allowed to cool to ambient lab conditions (23 ± 2 °C) for 90 ± 30 minutes, at which point the polymer composition is removed from the PTFE bowl and placed between 2 sheets of siliconised paper (such as product number 114918, Mondi Group, Hilm, Austria, or equivalent). A metal shim 500 ± 30 µm in thickness is used in the heated press as a spacer to obtain a film thickness of 500 µm when pressed with a heated press at 90 °C for 60 seconds at a pressure sufficient to form a polymeric film. If 90 °C is insufficient to press a uniform flat film, a temperature approximately 10 ± 5 °C below the melting point of the sample material composition such that the sample material composition is in a semi-solid state is used. The film is stored at least 120 hours in the laboratory at 23 ± 2 °C prior to testing. From the film individual specimens for measurement are punched with a sample cutter to the final specimen dimensions of 20.0 mm by 10.0 mm by 500 µm.

### Measurement

To secure the film to the cylinders of the EVF, the cylinders are heated to 50 °C for 90 ± 30 s in the forced-convection oven of the rheometer. After opening the oven, a specimen of polymer composition is briefly pressed onto the cylinders of the EVF to secure it to the cylinder surface. The specimen is placed perpendicular to the axis of rotation of the cylinders.

The specimen mounted on the EVF is then placed in the forced convection oven of the rheometer for thermal conditioning and is kept isothermal at 23 ± 1 °C for 300 ± 10 s. After this time has elapsed, the specimen is mechanically conditioned. To mechanically condition the specimen, the torque transducer is zeroed, and the sample is put under a pre-stretch rate of 0.001 s⁻¹ for 0.30 s and then allowed to relax for 60 s (in this method, all strain is expressed in terms of Hencky strain, also known as "true strain" or "logarithmic strain.").

The measurement is performed in the FCO oven at 23 °C ± 0.5 °C. The strain rate extension for the measurement is 1 s⁻¹, and the strain at maximum extension is 4.0. After measurement, the specimen is checked for rupturing. If it has ruptured, the location of the break is noted. If the rupture is approximately in the middle between the two cylinders of the EVF, the data collected are deemed acceptable. Otherwise, if the polymeric film break is at or close to the rotating cylinders, the results are discarded, and the measurement performed again on a replicate specimen.

### Analysis

For the extensional stress calculation, a constant volume is assumed. From the raw torque versus angular displacement data recorded by the rheometer, extensional stress (in megapascals, or MPa) versus Hencky strain data are calculated. The data are plotted in semilogarithmic fashion with Hencky strain on the abscissa (linear scale) and extensional stress on the ordinate (logarithmic scale). A linear range is sought in this plot. If a linear range above a strain of 0.3 can be identified and this range can be fit with a positive slope with an R² value of 0.98 or greater, the value of the fitted line at a Hencky strain of zero (that is, the y-intercept), is defined as the Yield Stress, which is reported in MPa to the nearest kilopascal. Otherwise, the maximum value of extensional stress recorded during the measurement is reported as the Yield Stress, again reported in MPa to the nearest kilopascal.

The extensional stress (MPa) versus Hencky strain data calculated above are again plotted, but this time in linear fashion with Hencky strain on the abscissa (linear axis) and extensional stress on the ordinate (linear axis). The integral of extensional stress with strain (that is, the area under the extensional stress curve as a function of strain) is calculated from a strain of zero to the strain at which the sample ruptured (or, in the case it did not rupture during the measurement, to a strain of 4.0) and is reported as the Toughness, which is reported in units of megajoules per cubic meter, or MJ m⁻³.

### Contact Angle Method

The Contact Angle Method is used to measure the contact angle of deionized water on a thin film of hotmelt adhesive after 24 hours ("Fresh film contact angle) and accelerated ageing ("Aged film contact angle). Contact angles of deionized water (resistivity 18.2 MΩ or greater at 25 °C) on sample adhesive substrate are determined using the sessile-drop approach generally described in ASTM D7490-13 using a goniometer and appropriate image acquisition system. The goniometer stage is leveled, and the image acquisition system is configured so as to capture toward the center of the stage, along an axis that is in the plane of the stage. The image acquisition system is further configured such that a droplet delivered to the center of the stage is in focus. Contact-angle analysis is carried out under a laboratory environment of 23 ± 2 °C and 40 ± 10% relative humidity.

A continuous adhesive film (at least 50 micrometer thick) is coated on a smooth substrate (e.g. silicone-coated paper) by a hotmelt coater at 170°C (adapt temperature if needed). The film is then cooled down to ambient lab conditions (21-23°C, 40%-60% RH). The contact angle testing is conducted on the adhesive surface 24 hour after the adhesive coating making.

Five specimen locations from one or more like regions of a sample adhesive are analyzed. Sufficiently many like sample adhesive regions required to provide five specimens 1 cm × 1 cm in dimension are selected. The five specimens are excised from the sample adhesive regions. Each specimen is analyzed by first placing it on the leveled goniometer stage. A flat-tipped, 14-gauge needle (outer diameter 2.11 mm) is then used to deliver a 25.0 ± 0.05 µL droplet of deionized water on the center of the specimen. An image of the droplet is captured 5.0 seconds after delivery. Tangent lines are drawn at both horizontal extrema of the imaged droplet, and the angle containing the droplet-substrate interface between each tangent line and the horizontal substrate is measured. The arithmetic mean of these two angles is the contact angle for that specimen and is recorded to the nearest 0.1°. The contact angles for the five specimens are determined in this way. The arithmetic mean of the five specimen contact angles is calculated and recorded as the Fresh Film Contact Angle.

The Aged Film Contact Angle is obtained in similar manner with the difference is that the adhesive film is aged at ambient pressure and at 60 ± 3 °C for 16 days. Subsequently, the adhesive film is kept at 23 ± 2 °C and 40 ± 10% relative humidity for 24 hours immediately prior to contact-angle analysis as indicated above.

## Claims

1. A fibrous layer (28) having a first side and an opposed second side, wherein the fibrous layer comprises a hotmelt composition (32) that at least partially coats and binds the fibers (12, 16) of the fibrous layer on the surface of at least one of the first side and second side, wherein the hotmelt composition comprises at least 50% by weight of the hotmelt composition of a metallocene-catalyzed polymer, wherein the metallocene-catalyzed polymer is a propylene-based copolymer.

2. The fibrous layer according to claim 1, wherein the metallocene-catalyzed polymer has a peak molecular weight in the range of from 10,000 g/mol to 130,000 g/mol, as measured by the method of the description.

3. The fibrous layer according to any of the preceding claims, wherein the hotmelt composition comprises less than 10 % by weight of tackifier.

4. The fibrous layer according to any of the preceding claims, wherein the hotmelt composition has a storage modulus (G') at 37 °C of from about 3 MPa to 40 MPa, as measured by the Oscillatory Rheometry Test Method.

5. The fibrous layer according to any of the preceding claims, wherein the hotmelt composition has a Toughness at 23 °C at least 10 MJ m⁻³, in particular at least 15 MJ m⁻³ as measured by the Extensional Test Method described herein.

6. The fibrous layer according to any of the preceding claims, wherein the fibrous layer comprise cellulose fibers (12) and optionally spunmelt fibers (16) intermixed with the cellulose fibers.

7. The fibrous layer according to any of the preceding claims, wherein the fibrous layer comprises at least 30% by weight, preferably at least 50% by weight, of cellulose fibers.

8. The fibrous layer according to any of the preceding claims, wherein the hotmelt composition comprises a hydrophilic melt additive.

9. The fibrous layer according to any of the preceding claims, wherein the hotmelt composition has a viscosity at 170°C ranging from 100 mPa.s to 10,000 mPa.s, as measured by the Viscosity Test Method disclosed herein.

10. A method for making a fibrous layer (28) according to any of the preceding claims, comprising the steps of:
a) providing a fibrous layer having a first side and an opposed second side,
b) spraying a molten hotmelt composition on the surface of the first side and/or second side of the fibrous layer; wherein the hotmelt composition coats and binds the fibers at the surface of the fibrous layer as the hotmelt composition solidifies.

11. Use of a hotmelt composition as indicated in any of the claims 1-9 on the surface of a fibrous layer to reduce fiber dusting of the fibrous layer.

12. A laminate (40) comprising a substrate layer (2) and a fibrous layer (28) according to any of the preceding claims 1-9.

13. An absorbent article (41) comprising a fibrous layer (28) or a laminate (40) according to any of the preceding claims 2-9 or 12.

14. An absorbent article (41) according to claim 13, wherein the fibrous layer or laminate is disposed between a topsheet (42) and an absorbent core (46).

## Patentansprüche

1. Faserschicht (28), die eine erste Seite und einer gegenüberliegenden zweite Seite aufweist, wobei die Faserschicht eine Hotmelt-Zusammensetzung (32), die die Fasern (12, 16) der Faserschicht auf der Oberfläche von wenigstens einer der ersten und der zweiten Seite wenigstens teilweise beschichtet und bindet, umfasst, wobei die Hotmelt-Zusammensetzung wenigstens zu 50 Gew.-% der Hotmelt-Zusammensetzung ein metallocenkatalysiertes Polymer umfasst, wobei das metallocenkatalysierte Polymer ein Copolymer auf Propylenbasis ist.

2. Faserschicht nach Anspruch 1, wobei das metallocenkatalysierte Polymer ein Peak-Molekulargewicht in dem Bereich von 10.000 g/mol bis 130.000 g/mol aufweist, wie gemessen durch das Verfahren der Beschreibung.

3. Faserschicht nach einem der vorstehenden Ansprüche, wobei die Hotmelt-Zusammensetzung weniger als zu 10 Gew.-% Klebrigmacher umfasst.

4. Faserschicht nach einem der vorstehenden Ansprüche, wobei die Hotmelt-Zusammensetzung einen Speichermodul (G') bei 37 °C von etwa 3 MPa bis 40 MPa aufweist, wie gemessen durch das Oszillationsrheometrieprüfverfahren.

5. Faserschicht nach einem der vorstehenden Ansprüche, wobei die Hotmelt-Zusammensetzung eine Zähigkeit bei 23 °C von wenigstens 10 MJ/m⁻³ insbesondere wenigstens 15 MJ/m⁻³ aufweist, wie gemessen durch das Dehnprüfverfahren.

6. Faserschicht nach einem der vorstehenden Ansprüche, wobei die Faserschicht Cellulosefasern (12) und wahlweise Schmelzspinnfasern (16), die mit den Cellulosefasern vermischt sind.

7. Faserschicht nach einem der vorhergehenden Ansprüche, wobei die Faserschicht wenigstens zu 30 Gew.-%, vorzugsweise wenigstens zu 50 Gew.-%, Cellulosefasern umfasst.

8. Faserschicht nach einem der vorstehenden Ansprüche, wobei die Hotmelt-Zusammensetzung einen hydrophilen Schmelzzusatzstoff umfasst.

9. Faserschicht nach einem der vorstehenden Ansprüche, wobei die Hotmelt-Zusammensetzung eine Viskosität bei 170 °C aufweist, die von 100 mPa.s bis 10.000 mPa.s reicht, wie gemessen durch das Viskositätsprüfverfahren.

10. Verfahren zum Herstellen einer Faserschicht (28) nach einem der vorstehenden Ansprüche, umfassend die Schritte:
a) Bereitstellen einer Faserschicht, die eine erste Seite und eine gegenüberliegende zweite Seite aufweist,
b) Aufsprühen einer geschmolzenen Hotmelt-Zusammensetzung auf die Oberfläche der ersten Seite und/oder der zweiten Seite der Faserschicht; wobei die Hotmelt-Zusammensetzung die Fasern an der Oberfläche der Faserschicht beschichtet und bindet, während die Hotmelt-Zusammensetzung erstarrt.

11. Verwendung einer Hotmelt-Zusammensetzung nach einem der Ansprüche 1 bis 9 auf der Oberfläche einer Faserschicht, um ein Faserstäuben auf der Faserschicht zu verringern.

12. Laminat (40), umfassend eine Substratschicht (2) und eine Faserschicht (28) nach einem der vorstehenden Ansprüche 1 bis 9.

13. Absorptionsartikel (41), umfassend eine Faserschicht (28) oder ein Laminat (40) nach einem der vorhergehenden Ansprüche 2 bis 9 oder 12.

14. Absorptionsartikel (41) nach Anspruch 13, wobei die Faserschicht oder das Laminat zwischen einer Oberschicht (42) und einem Absorptionskern (46) eingerichtet ist.

## Revendications

1. Couche fibreuse (28) ayant un premier côté et un second côté opposé, dans laquelle la couche fibreuse comprend une composition thermofusible (32) qui revêt et lie au moins partiellement les fibres (12, 16) de la couche fibreuse sur la surface d'au moins l'un parmi le premier côté et le second côté, dans laquelle la composition thermofusible comprend au moins 50 % en poids de la composition thermofusible d'un polymère catalysé par métallocène, dans laquelle le polymère catalysé par métallocène est un copolymère à base de propylène.

2. Couche fibreuse selon la revendication 1, dans laquelle le polymère catalysé par métallocène a une masse moléculaire maximale dans la plage allant de 10 000 g/mol à 130 000 g/mol, telle que mesurée par le procédé de la description.

3. Couche fibreuse selon l'une quelconque des revendications précédentes, dans laquelle la composition thermofusible comprend moins de 10 % en poids d'agent conférant de l'adhésivité.

4. Couche fibreuse selon l'une quelconque des revendications précédentes, dans laquelle la composition thermofusible a un module de conservation (G') à 37 °C allant d'environ 3 MPa à 40 MPa, tel que mesuré par le procédé de test de rhéométrie oscillatoire.

5. Couche fibreuse selon l'une quelconque des revendications précédentes, dans laquelle la composition thermofusible a une ténacité à 23 °C d'au moins 10 MJ m⁻³, en particulier au moins 15 MJ m⁻³ telle que mesurée par le procédé de test d'extension décrit ici.

6. Couche fibreuse selon l'une quelconque des revendications précédentes, dans laquelle la couche fibreuse comprend des fibres de cellulose (12) et facultativement des fibres filées par fusion (16) entremêlées avec les fibres de cellulose.

7. Couche fibreuse selon l'une quelconque des revendications précédentes, dans laquelle la couche fibreuse comprend au moins 30 % en poids, de préférence au moins 50 % en poids, de fibres de cellulose.

8. Couche fibreuse selon l'une quelconque des revendications précédentes, dans laquelle la composition thermofusible comprend un additif de fusion hydrophile.

9. Couche fibreuse selon l'une quelconque des revendications précédentes, dans laquelle la composition thermofusible a une viscosité à 170 °C allant de 100 mPa.s à 10 000 mPa.s, telle que mesurée par le procédé de test de viscosité décrit ici.

10. Procédé permettant de fabriquer une couche fibreuse (28) selon l'une quelconque des revendications précédentes, comprenant les étapes consistant à :
a) fournir une couche fibreuse ayant un premier côté et un second côté opposé,
b) pulvériser une composition thermofusible fondue sur la surface du premier côté et/ou du second côté de la couche fibreuse ; dans lequel la composition thermofusible revêt et lie les fibres au niveau de la surface de la couche fibreuse à mesure que la composition thermofusible se solidifie.

11. Utilisation d'une composition thermofusible telle qu'indiquée dans l'une quelconque des revendications 1 à 9 sur la surface d'une couche fibreuse pour réduire un poudrage de fibres de la couche fibreuse.

12. Stratifié (40) comprenant une couche de substrat (2) et une couche fibreuse (28) selon l'une quelconque des revendications 1 à 9 précédentes.

13. Article absorbant (41) comprenant une couche fibreuse (28) ou un stratifié (40) selon l'une quelconque des revendications 2 à 9 ou 12 précédentes.

14. Article absorbant (41) selon la revendication 13, dans lequel la couche fibreuse ou le stratifié est disposé entre une feuille de dessus (42) et une âme absorbante (46).
